# EUROPEAN PATENT APPLICATION

(11) **EP 4 831 178 A1**
(43) Date of publication of application: **28.10.2026**
(21) Application number: 24921188.9
(22) Date of filing: 22.04.2024
(51) Int. Cl.: C07D 487/04, A61K 31/4188, A61P 1/00, A61P 1/04, A61P 19/02, A61P 3/10, A61P 17/06, A61P 11/06, A61P 19/00, A61P 1/02, A61P 3/04, A61P 13/12, A61P 25/28, A61P 9/10

(54) **ARYL HETEROCYCLIC KV1.3 INHIBITOR, AND PREPARATION METHOD THEREFOR AND USE THEREOF**

(30) Priority: 29.01.2024 CN 202410114929
(71) Applicant: Shanghai Deep Potential Technology Co., Ltd., Xuhui District, Shanghai 200000 (CN)
(72) Inventor: HUANG, Dandan, Shanghai 201203 (CN); WANG, Dongdong, Shanghai 201203 (CN); ZHANG, Xiaomin, Shanghai 201203 (CN); SUN, Weijie, Shanghai 201203 (CN)
(74) Representative: Huang, Liwei
(86) International application number: PCT/CN2024/089005
(87) International publication number: WO 2025/161144

(57) **Abstract**

The present invention provides a novel Kv1.3 channel (or Kv1.3) inhibitor, which can be used for preventing and/or treating Kv1.3 channel (or Kv1.3)-related diseases, including immune and inflammatory diseases, such as multiple sclerosis, inflammatory bowel disease, ulcerative colitis, Crohn's disease, rheumatoid arthritis, type 1 diabetes, psoriasis and asthma, spondylitis and periodontitis; and obesity, type 2 diabetes, renal fibrosis, Alzheimer's disease, and ischemic stroke.

## Description

### TECHNICAL FIELD

The present invention relates to a Kv1.3 inhibitor, a method for preparing same, and use thereof. The inhibitor and a pharmaceutical composition comprising same can be used for preventing and/or treating Kv1.3-associated diseases or disorders.

### BACKGROUND

Kv1.3 channel (or Kv1.3) is a voltage-gated potassium channel (Kv) that is highly expressed on macrophages, microglia, and effector memory T (TEM) cells. The Kv1.3 channel can regulate cellular membrane potential and thus indirectly influence calcium signaling in TEM cells. TEM cells mediate a variety of immune and inflammatory diseases, such as multiple sclerosis, inflammatory bowel disease, rheumatoid arthritis, type 1 diabetes, psoriasis, asthma, and the like.

Autoimmune diseases are a large group of diseases caused by the body's own immune system mistakenly attacking normal cells and tissues. Current therapeutic regimens are typically anti-inflammatory or immunosuppressive medications, which can only relieve symptoms and are associated with serious toxic side effects. Studies have demonstrated a close etiological link between TEM cells and autoimmune diseases (Proceedings of the National Academy of Sciences, 103, 46 (2006): 17414-17419). Selective inhibition of TEM cell functions offers a therapeutic strategy for autoimmune diseases while avoiding impairment of protective immune responses and reducing treatment-induced side effects. TEM cells abundantly express Kv1.3 and rely on it to fulfill their cellular functions. Therefore, selective Kv1.3 inhibitors hold promise as low-toxicity, high-efficiency therapeutic drugs for autoimmune diseases.

Dalazatide (ShK-186), a derivative (obtained by derivatization with unnatural amino acids) of a Kv1.3-inhibitory peptide toxin isolated from scorpion and sea anemone, has shown a certain therapeutic effect in Phase Ib clinical trials for psoriasis treatment. However, the application of such molecules is limited due to the poor subtype selectivity and the lack of oral bioavailability of peptidic molecules. Therefore, the development of highly selective, orally bioavailable small-molecule Kv1.3 inhibitors has great social and market value.

### SUMMARY

The present invention provides a novel Kv1.3 channel (or Kv1.3) inhibitor which can be used for preventing and/or treating Kv1.3 channel (or Kv1.3)-associated diseases.

According to one aspect of the invention, provided is a compound of Formula (A) or a stereoisomer thereof, or a pharmaceutically acceptable salt or deuterated form thereof: 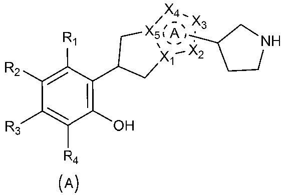 wherein
X1, X2, X3, X4, and X5 are each independently selected from C and N, and two of them are N, such that A is a 5-membered heteroaryl group containing two N atoms;
R1, R2, R3, and R4 are each independently selected from H, F, Cl, Br, CN, C1-4 linear or branched alkyl, C2-4 linear or branched alkenyl, C2-4 linear or branched alkynyl, C1-4 linear or branched alkyloxy, C2-4 linear or branched alkenyloxy, C2-4 linear or branched alkynyloxy, and C3-7 cycloalkyl.

In a preferred embodiment, in Formula (A):
R1, R2, R3, and R4 are each independently selected from H, F, Cl, Br, CN, C1-4 linear or branched alkyl, C2-4 linear or branched alkenyl, and C2-4 linear or branched alkynyl.

In a preferred embodiment, in Formula (A):
R1, R2, R3, and R4 are each independently selected from H, F, Cl, and Br.

In a preferred embodiment, in Formula (A):
R1 and R2 are each independently selected from F, Cl, and Br, and R3 and R4 are H.

In a preferred embodiment, in Formula (A):
X1 and X4 are N, and X2, X3, and X5 are C.

In a preferred embodiment, in Formula (A):
X2 and X3 are N, and X1, X4, and X5 are C.

In a preferred embodiment, in Formula (A):
X1 and X4 are N, and X2, X3, and X5 are C;
R1 and R2 are each independently selected from F, Cl, and Br, and R3 and R4 are H.

In a preferred embodiment, in Formula (A):
X2 and X3 are N, and X1, X4, and X5 are C;
R1 and R2 are each independently selected from F, Cl, and Br, and R3 and R4 are H.

According to one aspect of the invention, provided is a compound of Formula (I) or a stereoisomer thereof, or a pharmaceutically acceptable salt or deuterated form thereof: 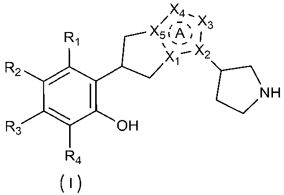 wherein
X1, X2, X3, X4, and X5 are each independently selected from C and N, and two of them are N, such that A is a 5-membered heteroaryl group containing two N atoms;
R1, R2, R3, and R4 are each independently selected from H, F, Cl, Br, CN, C1-4 linear or branched alkyl, C2-4 linear or branched alkenyl, C2-4 linear or branched alkynyl, C1-4 linear or branched alkyloxy, C2-4 linear or branched alkenyloxy, C2-4 linear or branched alkynyloxy, and C3-7 cycloalkyl.

In a preferred embodiment, in Formula (I):
R1, R2, R3, and R4 are each independently selected from H, F, Cl, Br, CN, C1-4 linear or branched alkyl, C2-4 linear or branched alkenyl, and C2-4 linear or branched alkynyl.

In a preferred embodiment, in Formula (I):
R1, R2, R3, and R4 are each independently selected from H, F, Cl, and Br.

In a preferred embodiment, in Formula (I):
X1 and X4 are N, and X2, X3, and X5 are C;
R1 and R2 are each independently selected from F, Cl, and Br, and R3 and R4 are H.

In a preferred embodiment, in Formula (I):
X2 and X3 are N, and X1, X4, and X5 are C;
R1 and R2 are each independently selected from F, Cl, and Br, and R3 and R4 are H.

According to one aspect of the invention, provided is a compound of Formula (II) or a stereoisomer thereof, or a pharmaceutically acceptable salt or deuterated form thereof: 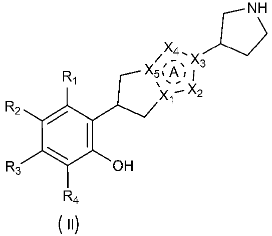 wherein
X1, X2, X3, X4, and X5 are each independently selected from C and N, and two of them are N, such that A is a 5-membered heteroaryl group containing two N atoms;
R1, R2, R3, and R4 are each independently selected from H, F, Cl, Br, CN, C1-4 linear or branched alkyl, C2-4 linear or branched alkenyl, C2-4 linear or branched alkynyl, C1-4 linear or branched alkyloxy, C2-4 linear or branched alkenyloxy, C2-4 linear or branched alkynyloxy, and C3-7 cycloalkyl.

In a preferred embodiment, in Formula (II):
R1, R2, R3, and R4 are each independently selected from H, F, Cl, Br, CN, C1-4 linear or branched alkyl, C2-4 linear or branched alkenyl, and C2-4 linear or branched alkynyl.

In a preferred embodiment, in Formula (II):
R1, R2, R3, and R4 are each independently selected from H, F, Cl, and Br.

In a preferred embodiment, in Formula (II):
X2 and X3 are N, and X1, X4, and X5 are C;
R1 and R2 are each independently selected from F, Cl, and Br, and R3 and R4 are H.

In a preferred embodiment, the compound of the invention is selected from the following compounds or pharmaceutically acceptable salts or deuterated forms thereof:

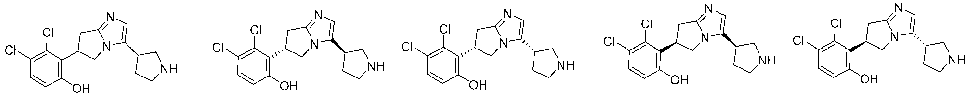

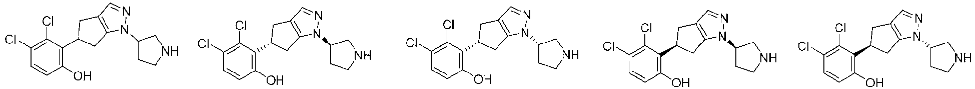

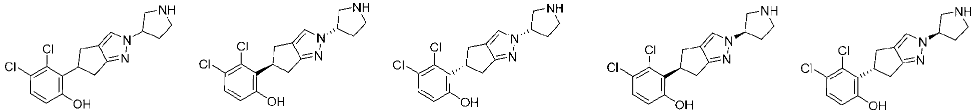

In the invention, although substituents are disclosed as groups or ranges, each group or range disclosed herein is intended to specifically cover every individual substituent encompassed therein. For example, the term "C1-4 alkyl" refers specifically to methyl (i.e., C1 alkyl), ethyl (i.e., C2 alkyl), propyl (i.e., C3 alkyl), and butyl (i.e., C4 alkyl) independently disclosed.

In the invention, examples of the "C3-7 cycloalkyl" include cyclopropyl, cyclobutyl, cyclopentyl, cyclohexyl, and cycloheptyl.

In the invention, examples of the "5-membered heteroaryl group containing two N atoms" include pyrazolyl and imidazolyl.

The compound of the invention may be asymmetric, for example, having one or more stereocenters. All stereoisomers, such as enantiomers and diastereomers, are included within the scope of the invention unless otherwise indicated. In the invention, compounds containing asymmetrically substituted carbon atoms may be resolved in optically active or racemic forms. Various methods for preparing optically active forms are known in the art, such as resolution of racemic mixtures or stereoselective synthesis.

The invention also includes a pharmaceutically acceptable salt of the compound. The pharmaceutically acceptable salt may be prepared by reacting the compound of the invention with a non-toxic inorganic or organic acid. Examples of the inorganic acids include hydrochloric acid, hydrobromic acid, hydroiodic acid, phosphoric acid, sulfuric acid, nitric acid, hydrogen sulfate, boric acid, hemisulfuric acid, and the like; and examples of the organic acids include formic acid, acetic acid, propionic acid, butyric acid, valeric acid, hexanoic acid, heptanoic acid, undecanoic acid, palmitic acid, stearic acid, oleic acid, oxalic acid, malonic acid, adipic acid, lactic acid, malic acid, maleic acid, hippuric acid, tartaric acid, citric acid, succinic acid, ascorbic acid, methanesulfonic acid, ethanesulfonic acid, benzenesulfonic acid, p-toluenesulfonic acid, benzoic acid, camphoric acid, camphorsulfonic acid, citric acid, fumaric acid, gluconic acid, galacturonic acid, dodecyl sulfuric acid, various amino acids, and the like.

The invention also includes a hydrate and a solvate of the compound.

The invention also includes all forms of the compound whose atoms are replaced by various isotopes. Isotopes include all atoms having the same atomic number but different mass numbers. For example, isotopes of hydrogen include deuterium.

The invention also includes a prodrug of the compound. The term "prodrug" refers to a compound obtained through structural modification of the compound, which has no or little activity in vitro, but releases the compound in patients via enzymatic or non-enzymatic conversion to exert its therapeutic effect.

It will be appreciated by those skilled in the art that the compound of the invention can be prepared by various methods already disclosed in the literature. The compound can be prepared by a reaction in a suitable solvent, and those skilled in the art of organic synthesis can readily select a suitable solvent that is essentially non-reactive with the reactants, intermediates, or products. The reaction may be conducted in a single solvent or a mixture of more than one solvent. The reaction for preparing the compound may be conducted at a suitable temperature, for example, a temperature between the freezing temperature of the solvent and the boiling point temperature of the solvent. The methods for preparing the compound involve the protection and deprotection of various chemical groups. Those skilled in the art of organic synthesis can readily determine whether protection and deprotection of chemical groups are required and select appropriate protecting groups. The reaction for preparing the compound may be monitored using any method known in the art, such as nuclear magnetic resonance spectroscopy, infrared spectroscopy, mass spectrometry, chromatography, and the like. Schemes 1 and 2 below illustrate synthetic routes that can be used to synthesize the compound of the invention, such as a compound having a structure of Formula I.

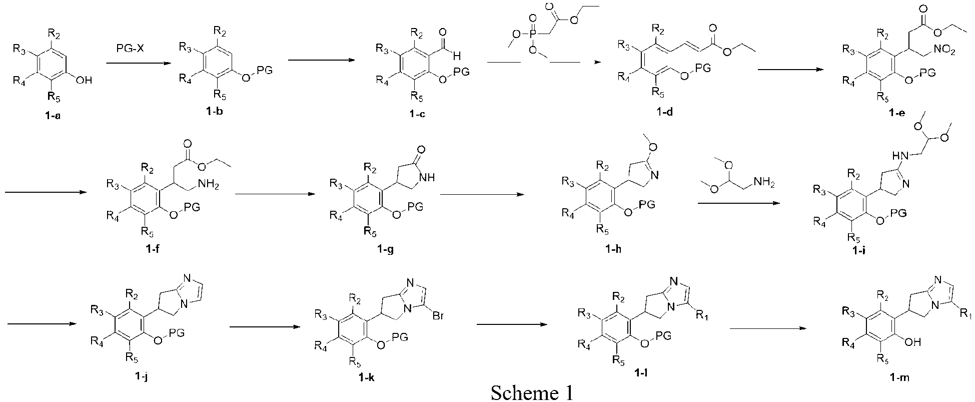

Compounds 1-a, 1-b, and 1-c, as shown in Scheme 1, can be prepared by any method known in the art and/or purchased commercially. As shown in Scheme 1, PG refers to a protecting group. Non-limiting examples of protecting groups include methyl, allyl, acetyl, tert-butoxycarbonyl, benzyl, or other protecting groups known in the art that are suitable as OH-protecting groups. Other substituents are defined herein. As shown in Scheme 1, 1-a can undergo a two-step reaction sequence involving phenolic hydroxyl protection followed by formylation to give 1-c. 1-c is subjected to the Wittig reaction, nitromethane substitution, nitro reduction, and intramolecular cyclization to give 1-g, which is then reacted with trimethyloxonium tetrafluoroborate to give Intermediate 1-h. 1-h undergoes a two-step reaction sequence involving substitution with aminoacetaldehyde dimethyl acetal followed by cyclization to give 1 -j, which is then subjected to a bromination reaction to give 1-k. 1-k can be converted to 1-l via the Suzuki reaction or the Stille reaction, or through substitution with a hydroxyl- or amino-containing compound; subsequently, 1-l is subjected to deprotection to give the final product 1-m.

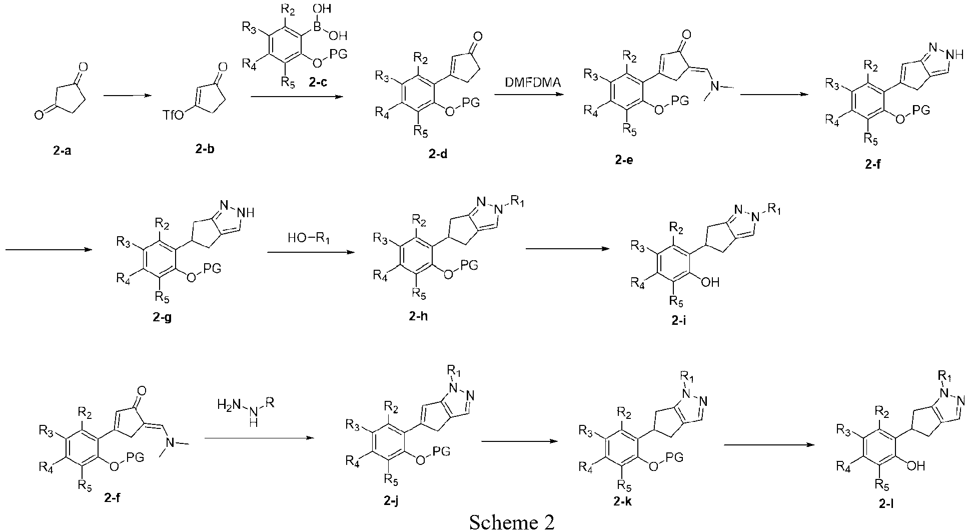

Compound 2-c, as shown in Scheme 2, can be prepared by any method known in the art and/or purchased commercially. As shown in Scheme 2, 2-a is reacted with trifluoromethanesulfonic anhydride to give 2-b, which then undergoes the Suzuki reaction with 2-c to give 2-d. 2-d is reacted with DMFDMA to give 2-e, which then undergoes a two-step reaction sequence involving cyclization with hydrazine hydrate followed by reduction to give 2-g. 2-g undergoes the Mitsunobu reaction or a substitution reaction to give 2-h, which is then deprotected to give the final product 2-i. Alternatively, intermediate 2-f can undergo cyclization with a substituted hydrazine to give 2-j, which then undergoes a two-step reaction sequence involving reduction followed by deprotection to give the final product 2-l.

The compound of the invention is capable of inhibiting Kv1.3. Therefore, according to another aspect of the invention, provided is a method for inhibiting Kv1.3 by using the compound.

According to another aspect of the invention, provided is a method for preventing and/or treating a Kv1.3-associated disease, comprising administering to an individual in need thereof a therapeutically and/or prophylactically effective amount of the compound or a pharmaceutical composition comprising said compound.

The Kv1.3-associated disease includes any disease directly and/or indirectly associated with the expression and/or activity of Kv1.3, for example, a disease that can be prevented and/or treated by inhibiting Kv1.3.

The Kv1.3-associated disease includes any of a group of immune and inflammatory diseases, such as multiple sclerosis, inflammatory bowel disease, ulcerative colitis, Crohn's disease, rheumatoid arthritis, type 1 diabetes, psoriasis and asthma, spondylitis, and periodontitis, as well as obesity, type 2 diabetes, renal fibrosis, Alzheimer's disease, ischemic stroke, and the like.

According to another aspect of the invention, provided is use of the compound in preparing a Kv1.3 inhibitor.

According to another aspect of the invention, provided is use of the compound in preparing a medicament for preventing and/or treating a Kv1.3-associated disease.

When the compound of the invention is used to prevent and/or treat a Kv1.3-associated disease, the compound may be administered in the form of a pharmaceutical composition. Therefore, according to another aspect of the invention, provided is a pharmaceutical composition comprising said compound and a pharmaceutically acceptable carrier.

It will be appreciated by those skilled in the art that the pharmaceutical composition of the invention can be prepared by various methods already disclosed in the literature. The compound or the pharmaceutical compositions may be administered via various routes, depending on whether local or systemic treatment is required and the area in need of treatment. For example, administration may be carried out orally, parenterally (e.g., by intravenous, intraarterial, subcutaneous, intraperitoneal, or intramuscular injection or infusion), intracranially (e.g., intrathecally or intracerebroventricularly), transdermally, ocularly, nasally, vaginally, rectally, or pulmonarily (e.g., by inhalation or insufflation of powders or aerosols).

For oral administration, the pharmaceutical composition is typically provided in the form of a tablet, a capsule, or a solution. The tablet may contain the compound or the pharmaceutically acceptable salt thereof and a pharmaceutically acceptable carrier. The carrier includes, but is not limited to, a diluent, a disintegrant, a binder, a lubricant, a colorant, or a preservative. The capsule includes a hard capsule and a soft capsule. For parenteral administration, the pharmaceutical composition may be administered by intravenous injection, intramuscular injection, or subcutaneous injection. It is usually provided as a sterile aqueous solution or suspension or lyophilized powder, with appropriately adjusted pH and isotonicity.

The effective amount of the compound may be determined based on the specific therapeutic use, the mode of administration, and the condition of an individual in need thereof (e.g., a patient). Those skilled in the art are capable of determining the effective amount of the compound. A typical dose range is, for example, from 1 µg/kg/day to 1000 mg/kg/day.

When preventing and/or treating a Kv1.3-associated disease, the compound may be used in combination with one or more additional drugs. The additional drugs include, but are not limited to, prednisone, methylprednisolone, ocrelizumab, natalizumab, alemtuzumab, β-interferon, glatiramer, fingolimod, dimethyl fumarate, diroximel, teriflunomide, siponimod, cladribine, mesalamine, balsalazide, olsalazine, azathioprine, mercaptopurine, methotrexate, infliximab, adalimumab, golimumab, certolizumab, vedolizumab, ustekinumab, methotrexate, leflunomide, hydroxychloroquine, sulfasalazine, abatacept, adalimumab, anakinra, certolizumab, etanercept, golimumab, infliximab, rituximab, sarilumab, tocilizumab, baricitinib, tofacitinib, upadacitinib, insulin glulisine, insulin lispro, insulin aspart, insulin glargine, insulin detemir, insulin degludec, fluticasone, budesonide, mometasone furoate, beclomethasone, ciclesonide, montelukast, zafirlukast, zileuton, salmeterol, omalizumab, mepolizumab, benralizumab, reslizumab, etc.

### BRIEF DESCRIPTION OF THE DRAWINGS

Fig. 1 shows an ellipsoid plot of the three-dimensional molecular structure of compound (S)-4-(6-(allyloxy)-2,3-dichlorophenyl)pyrrolidin-2-one.
Fig. 2 shows an ellipsoid plot of the three-dimensional molecular structure of compound (R)-4-(6-(allyloxy)-2,3-dichlorophenyl)pyrrolidin-2-one.
Fig. 3 shows the NOESY spectrum of Example 3.
Fig. 4 shows the NOESY spectrum of Example 4.
Fig. 5 shows the NOESY spectrum of Example 5.

### DETAILED DESCRIPTION

The invention will be further illustrated with reference to the following specific examples. It will be appreciated that these examples are merely intended to illustrate the invention rather than limit the scope of the invention. In addition, it will be appreciated that various changes or modifications may be made by those skilled in the art after reading the contents described in the invention, and these equivalents also fall within the scope of the invention.

### Intermediate 1. 3-(6-(Allyloxy)-2,3-dichlorophenyl)-5-methoxy-3,4-dihydro-2H-pyrrole

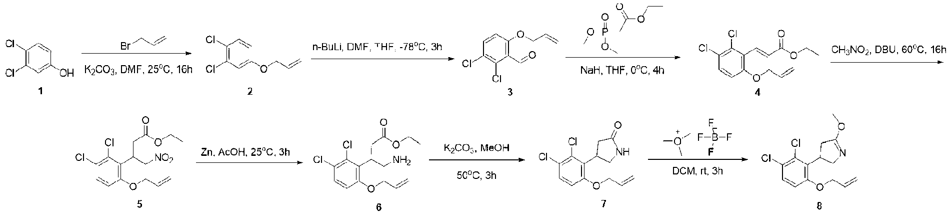

### Synthesis of 4-(allyloxy)-1,2-dichlorobenzene (2):

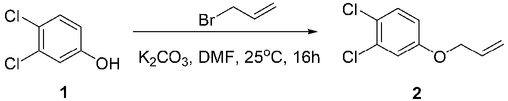

3,4-Dichlorophenol (120.0 g, 0.74 mol) and potassium carbonate (253.9 g, 1.84 mol) were mixed in N,N-dimethylformamide (1.5 L), and allyl bromide (106.9 g, 0.88 mol) was added in portions at 0 °C. The resulting mixture was stirred at room temperature for 16 h. After completion of the reaction, the mixture was diluted with water (1500 mL) and extracted twice with ethyl acetate (1500 mL). The organic phases were combined, washed with saturated brine (1500 mL), and dried over anhydrous sodium sulfate. After filtration, the filtrate was concentrated under reduced pressure, and the resulting residue was purified by silica gel column chromatography (petroleum ether:ethyl acetate = 20:1) to give a yellow solid (120.0 g, yield: 68%).

### Synthesis of 6-(allyloxy)-2,3-dichlorobenzaldehyde (3):

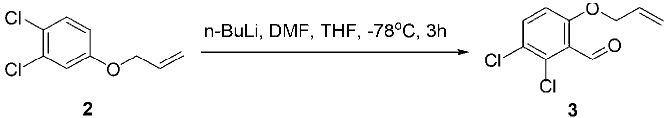

At -78 °C, 4-(allyloxy)-1,2-dichlorobenzene (120.0 g, 0.59 mol) was dissolved in tetrahydrofuran (1200 mL), and n-butyllithium (260.0 mL, 0.65 mol, 2.5 M in n-hexane) was added dropwise over a period of more than 30 min. Then, N,N-dimethylformamide (86.4 g, 1.18 mol) was added at the same temperature. The resulting mixture was further stirred at the same temperature for 3 h. After completion of the reaction, the reaction was quenched with a saturated aqueous ammonium chloride solution (1000 mL), and the resulting mixture was extracted twice with ethyl acetate (3000 mL). The organic phases were combined, washed with saturated brine (1500 mL), dried over anhydrous sodium sulfate, and then concentrated under reduced pressure. The residue was purified by silica gel column chromatography (petroleum ether:ethyl acetate = 15:1) to give a pale yellow solid (139.5 g, yield: 86%).

1H NMR (400 MHz, DMSO-d6) δ 10.36 (s, 1H), 7.83 (d, J = 9.1 Hz, 1H), 7.25 (d, J = 9.1 Hz, 1H), 6.13 - 5.96 (m, 1H), 5.46 (m, J = 17.3, 1.7 Hz, 1H), 5.31 (m, J = 10.6, 3.0, 1.4 Hz, 1H), 4.74 (dt, J = 4.9, 1.5 Hz, 2H).

### Synthesis of ethyl (E)-3-(6-(allyloxy)-2,3-dichlorophenyl)acrylate (4):

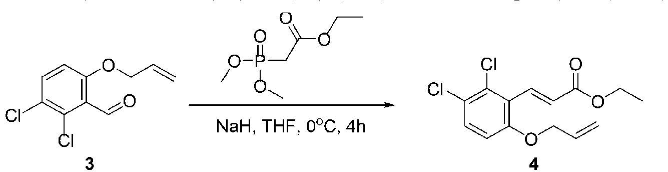

At 0 °C, sodium hydride (29.1 g, 0.73 mol, 60% in mineral oil) was added to a solution of ethyl 2-(dimethoxyphosphoryl)acetate (142.6 g, 0.73 mol) in tetrahydrofuran (2000 mL). The resulting mixture was stirred for 30 min before adding 6-(allyloxy)-2,3-dichlorobenzaldehyde (139.5 g, 0.61 mol), and then stirred at 0 °C for 4 h. After completion of the reaction, the mixture was diluted with water (2000 mL) and extracted with ethyl acetate (4000 mL). The organic phase was washed with saturated brine (2000 mL), dried over anhydrous sodium sulfate, and concentrated under reduced pressure. The resulting residue was purified by silica gel column chromatography (petroleum ether:ethyl acetate = 5:1) to give a yellow solid (170.0 g, yield: 79%).

1H NMR (400 MHz, DMSO-d6) δ 7.84 (d, J = 16.2 Hz, 1H), 7.64 (d, J = 9.1 Hz, 1H), 7.17 (d, J = 9.2 Hz, 1H), 6.81 (d, J = 16.2 Hz, 1H), 6.13 - 6.00 (m, 1H), 5.47 - 5.25 (m, 2H), 4.74 (dt, J = 5.2, 1.5 Hz, 2H), 4.21 (q, J = 7.1 Hz, 2H), 1.26 (t, J = 7.1 Hz, 3H).

### Synthesis of ethyl 3-(6-(allyloxy)-2,3-dichlorophenyl)-4-nitrobutanoate (5):

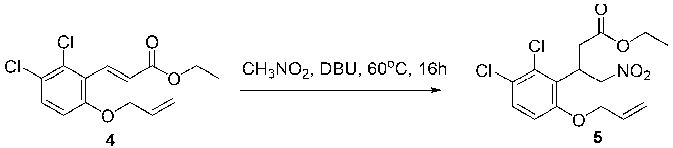

At room temperature, ethyl (E)-3-(6-(allyloxy)-2,3-dichlorophenyl)acrylate (170.0 g, 0.56 mol) and 1,8-diazabicyclo[5.4.0]undec-7-ene (103.1 g, 0.68 mol) were added to a reaction flask containing nitromethane (2000 mL). The reaction mixture was stirred at 60 °C for 16 h. After completion of the reaction, the mixture was diluted with water (1000 mL) and extracted with ethyl acetate (3000 mL). The resulting organic phase was washed with saturated brine (1000 mL), dried over anhydrous sodium sulfate, and then concentrated under reduced pressure. The residue was purified by silica gel column chromatography (petroleum ether:ethyl acetate = 5:1) to give a pale yellow oily matter (174.0 g, yield: 72%).

1H NMR (400 MHz, DMSO-d6) δ 7.53 (d, J = 9.0 Hz, 1H), 7.06 (d, J = 9.1 Hz, 1H), 6.07 (ddd, J = 15.9, 10.6, 5.2 Hz, 1H), 5.41 (d, J = 17.2 Hz, 1H), 5.33 (dd, J = 10.6, 1.1 Hz, 1H), 5.00 (ddd, J = 19.0, 13.0, 7.5 Hz, 2H), 4.69 (d, J = 4.4 Hz, 3H), 4.04 - 3.91 (m, 2H), 2.89 (dq, J = 17.6, 8.7 Hz, 2H), 1.06 (t, J = 7.1 Hz, 3H).

### Synthesis of ethyl 3-(6-(allyloxy)-2,3-dichlorophenyl)-4-aminobutanoate (6):

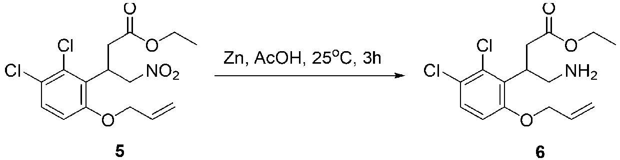

At room temperature, ethyl 3-(6-(allyloxy)-2,3-dichlorophenyl)-4-nitrobutanoate (174.0 g, 0.48 mol) and zinc powder (314.2 g, 4.80 mol) were added to a reaction flask containing glacial acetic acid (2000 mL). The mixture was stirred at 25 °C for 3 h. After completion of the reaction, the mixture was filtered, and the filter cake was washed twice with water and ethyl acetate. The filtrate was collected and concentrated under reduced pressure to give a crude product (200.0 g), which was directly used in the next step without purification.

LCMS [M+H]+: 332.1.

### Synthesis of 4-(6-(allyloxy)-2,3-dichlorophenyl)pyrrolidin-2-one (7):

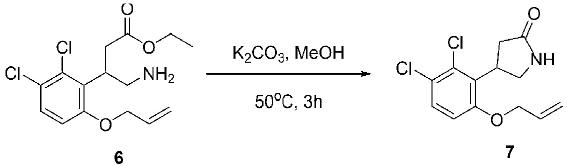

The crude product of ethyl 3-(6-(allyloxy)-2,3-dichlorophenyl)-4-aminobutanoate (200.0 g, 0.45 mol) and potassium carbonate (311.5 g, 2.26 mol) were added to methanol (2000 mL), and the mixture was stirred at 50 °C for 3 h. After completion of the reaction, the mixture was diluted with water (1000 mL) and extracted three times with ethyl acetate (3000 mL). The organic phases were combined and then dried over anhydrous sodium sulfate. After filtration, the filtrate was concentrated under reduced pressure. The residue was purified by silica gel column chromatography (petroleum ether:ethyl acetate = 5:1) to give a white solid (105.0 g, yield: 69%).

LCMS [M+H]+: 286.0.

1H NMR (400 MHz, DMSO-d6) δ 7.75 (s, 1H), 7.51 (d, J = 9.0 Hz, 1H), 7.08 (d, J = 9.0 Hz, 1H), 6.01 (ddt, J = 17.2, 10.4, 5.0 Hz, 1H), 5.44 - 5.35 (m, 1H), 5.26 (dd, J = 10.6, 1.5 Hz, 1H), 4.64 (dd, J = 3.6, 1.4 Hz, 2H), 4.33 (dd, J = 18.4, 9.2 Hz, 1H), 3.43 (dt, J = 16.7, 8.9 Hz, 2H), 2.54 (dd, J = 16.7, 9.0 Hz, 1H), 2.36 (dd, J = 16.5, 10.7 Hz, 1H).

### Synthesis of 3-(6-(allyloxy)-2,3-dichlorophenyl)-5-methoxy-3,4-dihydro-2H-pyrrole (8):

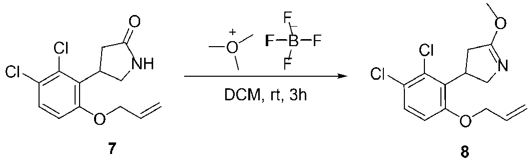

4-(6-(Allyloxy)-2,3-dichlorophenyl)pyrrolidin-2-one (500.0 mg, 1.75 mmol) was dissolved in dichloromethane (5 mL), and trimethyloxonium tetrafluoroborate (311.6 mg, 2.11 mmol) was added. The mixture was stirred at room temperature for 3 h in a nitrogen atmosphere. After completion of the reaction, the mixture was directly concentrated to dryness by rotary evaporation, and the resulting crude product was directly used in the next step without purification.

### Intermediate 2. (S)-3-(6-(Allyloxy)-2,3-dichlorophenyl)-5-methoxy-3,4-dihydro-2H-pyrrole

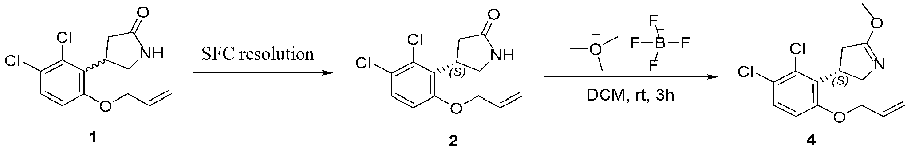

### Preparation of (S)-4-(6-(allyloxy)-2,3-dichlorophenyl)pyrrolidin-2-one (2):

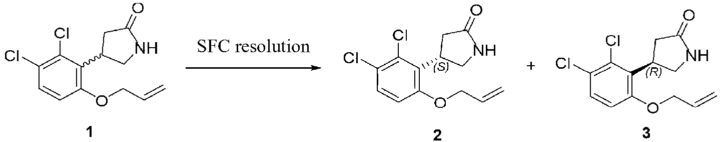

4-(6-(Allyloxy)-2,3-dichlorophenyl)pyrrolidin-2-one (10 g) was taken and purified by preparative chiral chromatography (gradient column: CHIRALPAK IC 250 mm × 4.6 mm, 5 µm, flow rate: 3 mL/min, gradient: 40% IPA (NH4OH 0.2%):60% CO2).

The first compound obtained from the SFC resolution was Compound 2 (4.5 g, yield: 45%) with a retention time (RT) of 4.51 min under the analytical SFC conditions.

LC-MS: [M+H]+: 286.0.

The second compound obtained from the SFC resolution was Compound 3 (4.3 g, yield: 43%) with an RT of 6.5 min under the analytical SFC conditions.

LC-MS: [M+H]+: 286.0.

### Single crystal X-ray diffraction detection analysis of Compounds 2 and 3

Preparation of single crystals: Single crystals of Compounds 2 and 3 were obtained through solvent diffusion crystallization under ethyl acetate/n-hexane conditions, with crystal growth proceeding at room temperature for 2 days.

### Single crystal detection instrument model: Bruker D8 Venture

### Instrument parameters:

| | |
|---|---|
| Light source: Cu target | X-ray: Cu-Kα (λ = 1.54178 Å) |
| Detector: CMOS area detector | Resolution: 0.86 Å |
| Current and voltage: 50 kV, 1.2 mA | Exposure time: 5 s |
| Current and voltage: 50 kV, 1.2 mA | Test temperature: 100(2) K |
| Area detector-to-sample distance: 40 mm | |

### Structure solution and refinement process:

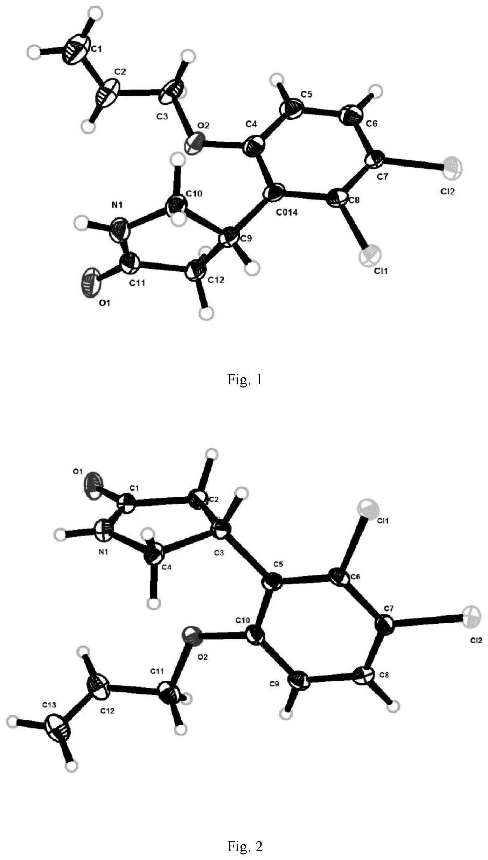

The diffraction data were integrated and reduced using the SAINT program, followed by empirical absorption correction using the SADABS program. The single crystal structure was solved by the direct method using SHELXT2014 and refined by the least square method. The hydrogen atom refinement process was conducted by isotropic calculation. The hydrogen atoms on C-Hs were positioned geometrically and refined using a riding model. The Flack parameter for the single crystal of Compound 2 was 0.087 (13), confirming the S-configuration at C9. The Flack parameter for the single crystal of Compound 3 was 0.073 (13), confirming the R-configuration at C3. The ellipsoid plots of the three-dimensional molecular structures of Compounds 2 and 3 are shown in Figs. 1 and 2, respectively. The crystal structure data for Compound 2 are shown in Table 1, and the crystal structure data for Compound 3 are shown in Table 2.

**Table 1. Crystal structure data for Compound 2**

| | |
|---|---|
| C13H13Cl2NO2 | Z = 4 |
| Mr = 286.14 | F(000) = 592 |
| Triclinic, P1 | Dx = 1.458 Mg m-3 |
| a = 8.1337 (3) Å | Cu Ka radiation, 1 = 1.54178 Å |
| b = 12.0320 (4) Å | Cell parameters from 9892 reflections |
| c = 14.0408 (5) Å | q = 3.9-63.7° |
| a = 71.569 (1)° | m = 4.43 mm-1 |
| b = 89.604 (1)° | T = 100 K |
| g = 89.392 (1)° | Block, colourless |
| V = 1303.53 (8) Å3 | 0.15 × 0.08 × 0.05 mm |

**Table 2. Crystal structure data for Compound 3**

| | |
|---|---|
| C13H13Cl2NO2 | Z = 4 |
| Mr = 286.14 | F(000) = 592 |
| Triclinic, P1 | Dx = 1.458 Mg m-3 |
| a = 8.1370 (2) Å | Cu Ka radiation, 1 = 1.54178 Å |
| b = 12.0239 (3) Å | Cell parameters from 9846 reflections |
| c = 14.0409 (3) Å | q = 3.3-63.7° |
| a = 71.591 (1)° | m = 4.43 mm-1 |
| b = 89.548 (1)° | T = 100 K |
| g = 89.353 (1)° | Block, colourless |
| V = 1303.34 (5) Å3 | 0.15 × 0.08 × 0.05 mm |

### Synthesis of (S)-3-(6-(allyloxy)-2,3-dichlorophenyl)-5-methoxy-3,4-dihydro-2H-pyrrole (4):

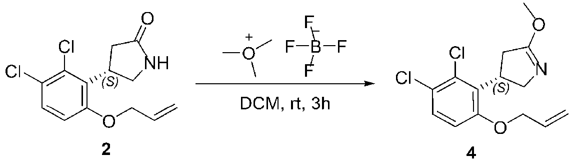

(S)-4-(6-(Allyloxy)-2,3-dichlorophenyl)pyrrolidin-2-one (1.0 g, 3.5 mmol) was dissolved in dichloromethane (10 mL), and trimethyloxonium tetrafluoroborate (623.4 mg, 4.23 mmol) was added. The mixture was stirred at room temperature for 3 h in a nitrogen atmosphere. After completion of the reaction, the mixture was directly concentrated to dryness by rotary evaporation, and the resulting crude product was directly used in the next step without purification.

### Examples 1-2: 3,4-Dichloro-2-((S)-3-((R)-pyrrolidin-3-yl)-6,7-dihydro-5H-pyrrolo[1,2-a]imidazol-6-yl)phenol and 3,4-dichloro-2-((S)-3-((S)-pyrrolidin-3-yl)-6,7-dihydro-5H-pyrrolo[1,2-a]imidazol-6-yl)phenol

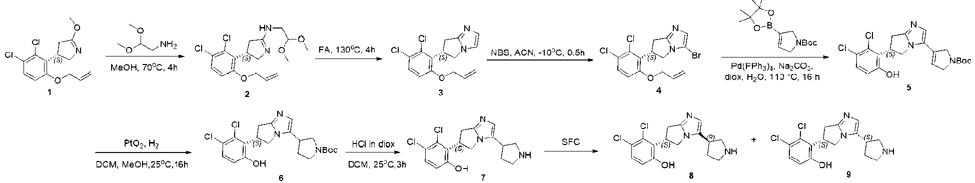

### Synthesis of (S)-3-(6-(allyloxy)-2,3-dichlorophenyl)-N-(2,2-dimethoxyethyl)-3,4-dihydro-2H-pyrrol-5-amine (2):

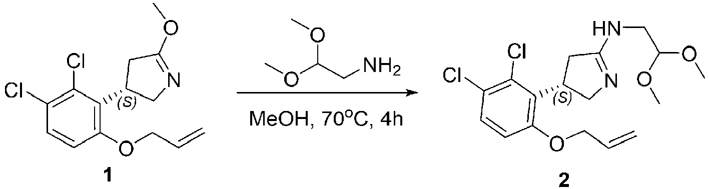

At room temperature, (S)-3-(6-(allyloxy)-2,3-dichlorophenyl)-5-methoxy-3,4-dihydro-2H-pyrrole (10.0 g, 33.33 mmol) and 2,2-dimethoxyethan-1-amine (4.2 g, 39.95 mmol) were added to methanol (100.0 mL). The resulting solution was stirred at 70 °C for 4 h. After completion of the reaction, the reaction mixture was concentrated under reduced pressure, and the resulting crude product was directly used in the next step without purification.

LC-MS: [M+H]+: 373.0.

### Synthesis of (S)-6-(6-(allyloxy)-2,3-dichlorophenyl)-6,7-dihydro-5H-pyrrolo[1,2-a]imidazole (3):

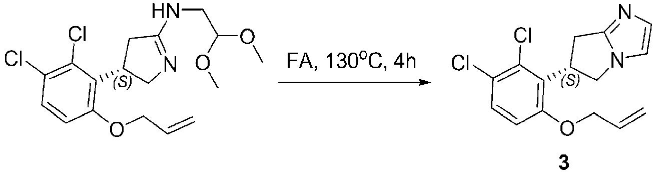

At room temperature, the crude product resulting from the previous step was dissolved in formic acid (100 mL). Then, the resulting mixture was stirred at 130 °C for 4 h. After completion of the reaction, the formic acid was removed by concentration under reduced pressure, and the resulting residue was diluted with a saturated aqueous sodium bicarbonate solution (200.0 mL) and extracted 3 times with ethyl acetate (500.0 mL). The organic phases were combined, washed with saturated aqueous sodium chloride (200.0 mL), dried over anhydrous sodium sulfate, and then concentrated under reduced pressure. The residue was separated by silica gel column chromatography (dichloromethane:methanol = 20:1) to give the target product (8.1 g, 66%).

LC-MS: [M+H]+: 308.9.

### Synthesis of (S)-6-(6-(allyloxy)-2,3-dichlorophenyl)-3-bromo-6,7-dihydro-5H-pyrrolo[1,2-a]imidazole (4):

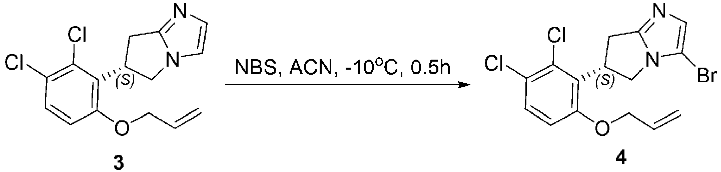

At -10 °C, (S)-6-(6-(allyloxy)-2,3-dichlorophenyl)-6,7-dihydro-5H-pyrrolo[1,2-a]imidazole (6.5 g, 0.021 mol) was dissolved in acetonitrile (65 mL), and then N-bromosuccinimide (3.4 g, 0.019 mol) was added in portions. Then, the mixture was stirred for 30 min with the temperature maintained. After completion of the reaction, the reaction was quenched with a sodium thiosulfate solution (100 mL), and the resulting mixture was extracted twice with ethyl acetate (200 mL). The organic phases were combined, washed with saturated brine (100 mL), dried over anhydrous sodium sulfate, and then concentrated under reduced pressure. The residue was purified by silica gel column chromatography (dichloromethane:methanol = 15:1) to give a pale yellow solid (2.4 g, yield: 25%).

LC-MS: [M+H]+: 386.7.

### Synthesis of tert-butyl (S)-3-(6-(2,3-dichloro-6-hydroxyphenyl)-6,7-dihydro-5H-pyrrolo[1,2-a]imidazol-3-yl)-2,5-dihydro-1H-pyrrol-1-carboxylate (5):

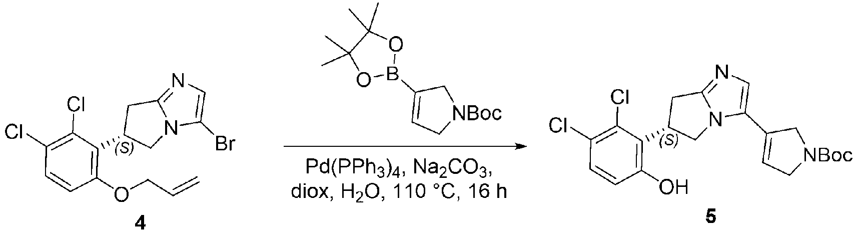

At room temperature, (S)-6-(6-(allyloxy)-2,3-dichlorophenyl)-3-bromo-6,7-dihydro-5H-pyrrolo[1,2-a]imidazole (370.0 mg, 0.95 mmol), tert-butyl 3-(4,4,5,5-tetramethyl-1,3,2-dioxaborolan-2-yl)-2,5-dihydro-1H-pyrrol-1-carboxylate (562.8 mg, 1.91 mmol), sodium carbonate (202.1 mg, 1.91 mmol), and tetrakis(triphenylphosphine)palladium(0) (110.2 mg, 0.09 mmol) were added to a reaction flask containing dioxane and water (10 mL, 1 mL). The mixture was stirred at 110 °C for 16 h. After completion of the reaction, the mixture was diluted by adding water (30 mL) and extracted twice with ethyl acetate (50 mL). The organic phases were combined, washed with saturated brine (50 mL), dried over anhydrous sodium sulfate, and then concentrated under reduced pressure. The residue was purified by silica gel column chromatography and eluted with dichloromethane: methanol = 10: 1 to give tert-butyl (S)-3-(6-(2,3-dichloro-6-hydroxyphenyl)-6,7-dihydro-5H-pyrrolo[1,2-a]imidazol-3-yl)-2,5-dihydro-1H-pyrrol-1-carboxylate in the form of an off-white solid (190 mg, yield: 38%).

LC-MS: [M+H]+: 435.7.

### Synthesis of tert-butyl 3-((S)-6-(2,3-dichloro-6-hydroxyphenyl)-6,7-dihydro-5H-pyrrolo[1,2-a]imidazol-3-yl)pyrrolidin-1-carboxylate (6):

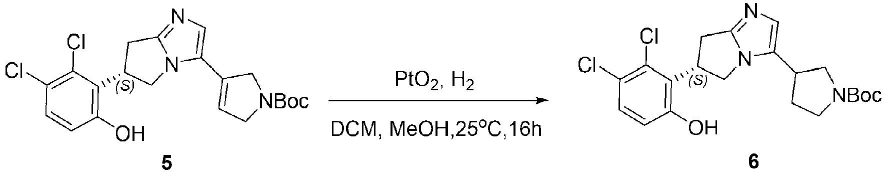

Tert-butyl (S)-3-(6-(2,3-dichloro-6-hydroxyphenyl)-6,7-dihydro-5H-pyrrolo[1,2-a]imidazol-3-yl)-2,5-dihydro-1H-pyrrol-1-carboxylate (180.0 mg, 0.41 mmol) and platinum dioxide (90.0 mg) were added to methanol and dichloromethane (5/5 mL), and the mixture was stirred at 25 °C for 16 h in a hydrogen atmosphere. After completion of the reaction, the mixture was filtered, and the filtrate was concentrated under reduced pressure to give the target product (120.0 mg, 66%), which was directly used in the next step without purification.

LC-MS: [M+H]+: 438.0.

### Synthesis of 3,4-dichloro-2-((6S)-3-(pyrrolidin-3-yl)-6,7-dihydro-5H-pyrrolo[1,2-a]imidazol-6-yl)phenol (7):

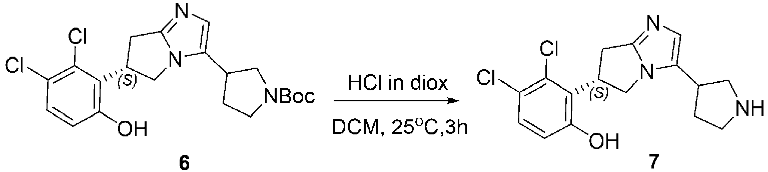

At room temperature, tert-butyl 3-((S)-6-(2,3-dichloro-6-hydroxyphenyl)-6,7-dihydro-5H-pyrrolo[1,2-a]imidazol-3-yl)pyrrolidin-1-carboxylate (120 mg) was added to dichloromethane (5.0 mL). A solution of hydrochloric acid in dioxane (1.0 mL) was slowly added to the reaction mixture, and the resulting mixture was stirred for 3 h. After completion of the reaction, the mixture was concentrated under reduced pressure. The resulting residue was subjected to preparative chromatography (column: Gemini-C18 150 × 21.2 mm, 5 µm, mobile phase: ACN-H2O (0.1% FA), gradient: 2-30) to give the target product (50.0 mg, 54%).

LC-MS: [M+H]+: 338.0.

### Preparation of 3,4-dichloro-2-((S)-3-((R)-pyrrolidin-3-yl)-6,7-dihydro-5H-pyrrolo[1,2-a]imidazol-6-yl)phenol and 3,4-dichloro-2-((S)-3-((S)-pyrrolidin-3-yl)-6,7-dihydro-5H-pyrrolo[1,2-a]imidazol-6-yl)phenol (8/9):

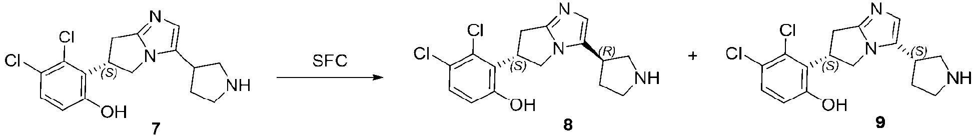

3,4-Dichloro-2-((6S)-3-(pyrrolidin-3-yl)-6,7-dihydro-5H-pyrrolo[1,2-a]imidazol-6-yl)phenol (50.0 mg, 0.14 mmol) was subjected to preparative chiral chromatography (column: CHIRALPAK AD-H 250 mm 20 mm, 5 µm, mobile phase: 40% EtOH (NH4OH 0.2%)) to give a pair of enantiomers.

The first compound obtained from the SFC resolution was designated as Example 1 (15.5 mg, yield: 31.0%), with a RT of 2.48 min under the analytical SFC conditions.

LC-MS: [M+H]+: 338.0.

1H NMR (400 MHz, DMSO-d6) δ 8.36 (s, 1H), 7.31 (d, J = 8.8 Hz, 1H), 7.06 (s, 1H), 6.81 (d, J = 8.8 Hz, 1H), 5.16 - 5.04 (m, 1H), 4.38 (t, J = 9.8 Hz, 1H), 4.28 (dd, J = 10.0, 7.5 Hz, 1H), 3.75 - 3.59 (m, 2H), 3.51 (ddd, J = 12.6, 8.2, 4.7 Hz, 1H), 3.39 (dt, J = 11.7, 8.4 Hz, 1H), 3.30 - 3.18 (m, 3H), 2.49 (td, J = 12.3, 7.0 Hz, 1H), 2.20 - 2.08 (m, 1H).

The second compound obtained from the SFC resolution was designated as Example 2 (14.1 mg, yield: 28.2%), with a RT of 4.37 min under the analytical SFC conditions.

LC-MS: [M+H]+: 338.0.

1H NMR (400 MHz, DMSO-d6) δ 8.37 (s, 1H), 7.31 (d, J = 8.8 Hz, 1H), 7.10 (s, 1H), 6.81 (d, J = 8.8 Hz, 1H), 5.09 (dd, J = 16.5, 8.1 Hz, 1H), 4.35 (dt, J = 17.1, 9.8 Hz, 2H), 3.81 - 3.59 (m, 2H), 3.52 (s, 1H), 3.39 (dd, J = 17.4, 9.2 Hz, 1H), 3.29 (dd, J = 10.7, 5.1 Hz, 3H), 2.49 (d, J = 6.0 Hz, 1H), 2.09 (dd, J = 12.4, 8.1 Hz, 1H).

### Examples 3-5: tert-Butyl (R)-3-((R)-5-(2,3-dichloro-6-methoxyphenyl)-5,6-dihydrocyclopenta[c]pyrazol-1(4H)yl)pyrrolidin-1-carboxylate, tert-butyl (R)-3-((S)-5-(2,3-dichloro-6-methoxyphenyl)-5,6-dihydrocyclopenta[c]pyrazol-1(4H)yl)pyrrolidin-1-carboxylate, and tert-butyl (3R)-3-(5-(2,3-dichloro-6-methoxyphenyl)-5,6-dihydrocyclopenta[c]pyrazol-2(4H)-yl)pyrrolidin-1-carboxylate

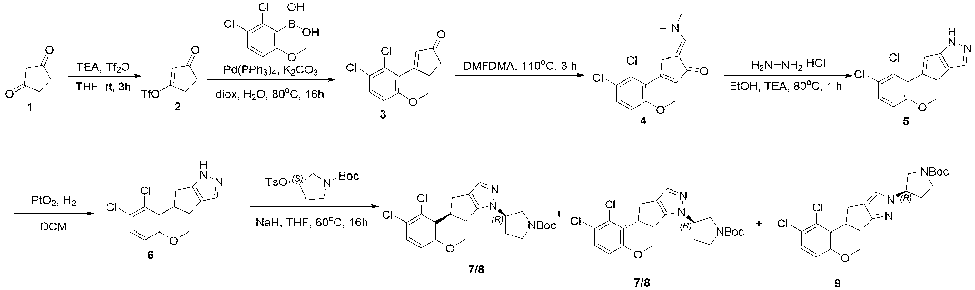

### Synthesis of 3-oxocyclopent-1-en-1-yl trifluoromethanesulfonate (2):

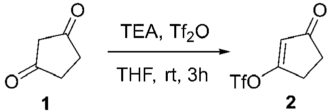

At 0 °C, triethylamine (20.5 g, 0.20 mol) was added to a solution of 1,3-cyclopentanedione (10.0 g, 0.10 mol) in tetrahydrofuran (150.0 mL). After 10 min of stirring, trifluoromethanesulfonic anhydride (34.5 g, 0.12 mol) was slowly added dropwise to the mixture with the temperature maintained at 0 °C. After completion of the dropwise addition, the mixture was warmed to room temperature and stirred for 3 h. After completion of the reaction as detected by TLC, the solvent of the mixture was removed by concentration under reduced pressure at 30 °C. The residue was wet-loaded and eluted with an eluent (petroleum ether:ethyl acetate = 20:1). The resulting product was concentrated under reduced pressure at 30 °C to remove most of the solvent, and the residue was directly used in the next step.

### Synthesis of 3-(2,3-dichloro-6-methoxyphenyl)cyclopent-2-en-1-one (3):

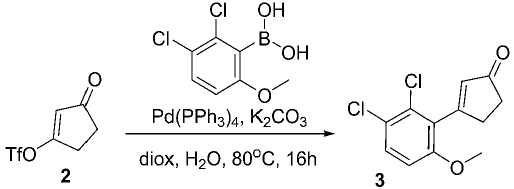

3-Oxocyclopent-1-en-1-yl trifluoromethanesulfonate (20.0 g, 0.09 mol), potassium carbonate (16.2 g, 0.12 mol), and tetrakis(triphenylphosphine)palladium(0) (5.2 g, 0.004 mol) were added to a mixed solution of (2,3-dichloro-6-methoxyphenyl)boronic acid (10.0 g, 0.05 mol) in dioxane:water (5:1, 300 mL). The mixture was stirred at 80 °C for 16 h in a nitrogen atmosphere. After completion of the reaction, the mixture was diluted with water (100.0 mL) and extracted twice with ethyl acetate (100.0 mL). The organic phases were combined, washed with saturated brine (200.0 mL), and dried over anhydrous sodium sulfate. After filtration, the filtrate was concentrated under reduced pressure, and the resulting residue was purified by silica gel column chromatography (
petroleum ether:ethyl acetate = 3:1) to give the target product (4.0 g, yield: 34%).

LC-MS: [M+H]+: 257.0.

### Synthesis of (E)-3-(2,3-dichloro-6-methoxyphenyl)-5-((dimethylamino)methylene)cyclopent-2-en-1-one (4):

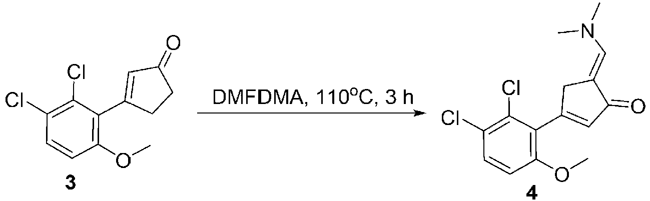

At room temperature, 3-(2,3-dichloro-6-methoxyphenyl)cyclopent-2-en-1-one (300.0 mg, 1.17 mmol) was added to a N,N-dimethylformamide dimethyl acetal solution (4.0 mL). The mixture was stirred at 110 °C for 3 h. After completion of the reaction, the mixture was directly concentrated by rotary evaporation to give a yellow oil (a mixture of crude product and starting material, 300.0 mg, yield: 50%), which was used directly in the next step.

LC-MS: [M+H]+: 312.0.

### Synthesis of 5-(2,3-dichloro-6-methoxyphenyl)-1,4-dihydrocyclopentadiene[c]pyrazole (5):

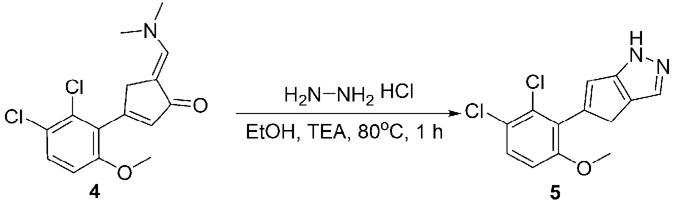

Triethylamine (0.25 mL) was added to a solution of (E)-3-(2,3-dichloro-6-methoxyphenyl)-5-((dimethylamino)methylene)cyclopent-2-en-1-one (300.0 mg, 0.96 mmol) and hydrazine hydrochloride (302.84 mg, 4.42 mmol) in ethanol (4.0 mL). The mixture was stirred at 80 °C for 1 h. After completion of the reaction, the filtrate was concentrated to dryness by rotary evaporation under reduced pressure, and the resulting crude product was purified by silica gel chromatography (dichloromethane:methanol = 30:1) to give a white solid (100.0 mg, yield: 37%).

LC-MS: [M+H]+: 281.0.

### Synthesis of 5-(2,3-dichloro-6-methoxyphenyl)-1,4,5,6-tetrahydrocyclopenta[c]pyrazole (6):

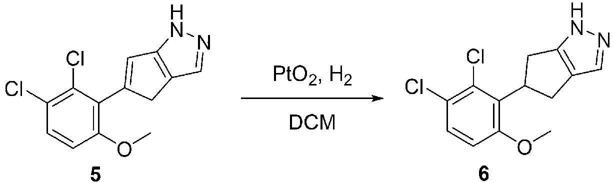

Platinum dioxide (701.1 mg, 3.09 mmol) was added to a solution of 5-(2,3-dichloro-6-methoxyphenyl)-1,4-dihydrocyclopentadiene[c]pyrazole (700.0 mg, 2.49 mmol) in dichloromethane (35.0 mL), and the mixture was stirred at room temperature for 7 days in a hydrogen atmosphere. After completion of the reaction, the mixture was filtered, and the resulting filtrate was concentrated under reduced pressure. The resulting crude product was purified by silica gel chromatography (dichloromethane:methanol = 40:1) to give a yellow solid (280.0 mg, yield: 40%).

LC-MS: [M+H]+: 283.0.

### Synthesis of tert-butyl (R)-3-((R)-5-(2,3-dichloro-6-methoxyphenyl)-5,6-dihydrocyclopenta[c]pyrazol-1(4H)yl)pyrrolidin-1-carboxylate, tert-butyl (R)-3-((S)-5-(2,3-dichloro-6-methoxyphenyl)-5,6-dihydrocyclopenta[c]pyrazol-1(4H)yl)pyrrolidin-1-carboxylate, and tert-butyl (3R)-3-(5-(2,3-dichloro-6-methoxyphenyl)-5,6-dihydrocyclopenta[c]pyrazol-2(4H)-yl)pyrrolidin-1-carboxylate (7/8/9):

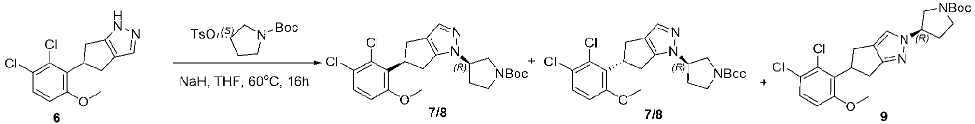

Sodium hydride (118.7 mg, 2.96 mmol, 60% in mineral oil) was added to a solution of 5-(2,3-dichloro-6-methoxyphenyl)-1,4,5,6-tetrahydrocyclopenta[c]pyrazole (280.0 mg, 0.99 mmol) and tert-butyl (S)-3-(tolyloxy)pyrrolidin-1-carboxylate (677.3 mg, 1.98 mmol) in tetrahydrofuran (28.0 mL), and the mixture was stirred at 60 °C for 16 h. After completion of the reaction, ice-water was added to quench the reaction, and the mixture was extracted twice with ethyl acetate. The organic phase was collected, washed with saturated brine, dried over anhydrous sodium sulfate, filtered, and concentrated to dryness by rotary evaporation in vacuo. The resulting crude product was separated by preparative chromatography (column: Gemini-C18 150 × 21.2 mm, 5µm; mobile phase: ACN---H2O (0.1% TFA), gradient: 70%-75%) to give three isomers.

The first compound obtained from preparative chromatography was designated as Example 3 (30.0 mg, yield: 7%), with an RT of 6.30 min in the preparative chromatography.

LC-MS: [M+H]+: 452.1.

1H NMR (400 MHz, DMSO-d6) δ 7.51 (d, J = 9.0 Hz, 1H), 7.18 (s, 1H), 7.08 (d, J = 9.1 Hz, 1H), 4.81 (dt, J = 14.3, 6.9 Hz, 2H), 3.76 (s, 3H), 3.69 - 3.54 (m, 2H), 3.41 - 3.29 (m, 2H), 2.97 (t, J = 9.8 Hz, 2H), 2.84 - 2.72 (m, 2H), 2.23 (tt, J = 13.0, 6.6 Hz, 2H), 1.34 (d, J = 12.2 Hz, 9H).

The second compound obtained from preparative chromatography was designated as Example 4 (40.0 mg, yield: 9%), with an RT of 6.65 min in the preparative chromatography.

LC-MS: [M+H]+: 452.1.

1H NMR (400 MHz, DMSO-d6) δ 7.52 (d, J = 8.9 Hz, 1H), 7.17 (s, 1H), 7.08 (d, J = 9.0 Hz, 1H), 4.80 (dd, J = 17.6, 9.0 Hz, 2H), 3.76 (d, J = 2.6 Hz, 3H), 3.67 (dd, J = 17.6, 10.6 Hz, 1H), 3.51 (dd, J = 11.2, 4.7 Hz, 1H), 3.43 (t, J = 7.5 Hz, 1H), 3.34 (dd, J = 13.3, 6.8 Hz, 1H), 3.06-2.93 (m, 2H), 2.78 (qd, J = 13.7, 8.9 Hz, 2H), 2.29 - 2.16 (m, 2H), 1.37 (d, J = 6.1 Hz, 9H).

The third compound obtained from preparative chromatography was designated as Example 5 (40.0 mg, yield: 9%), with an RT of 8.12 min in the preparative chromatography.

LC-MS: [M+H]+: 452.1.

1H NMR (400 MHz, DMSO-d6) δ 7.51 (d, J=9.0 Hz, 1H), 7.42 (d, J = 4.0 Hz, 1H), 7.07 (d, J = 9.0 Hz, 1H), 4.87 (d, J = 5.9 Hz, 1H), 4.60 (p, J = 9.1 Hz, 1H), 3.76 (s, 3H), 3.69 (dd, J = 10.2, 4.1 Hz, 1H), 3.54 - 3.43 (m, 2H), 3.37 (t, J = 7.1 Hz, 1H), 2.94 (ddd, J = 33.6, 14.5, 8.9 Hz, 2H), 2.78 (ddd, J = 14.4, 9.0, 5.6 Hz, 2H), 2.33 - 2.23 (m, 2H), 1.40 (s, 9H).

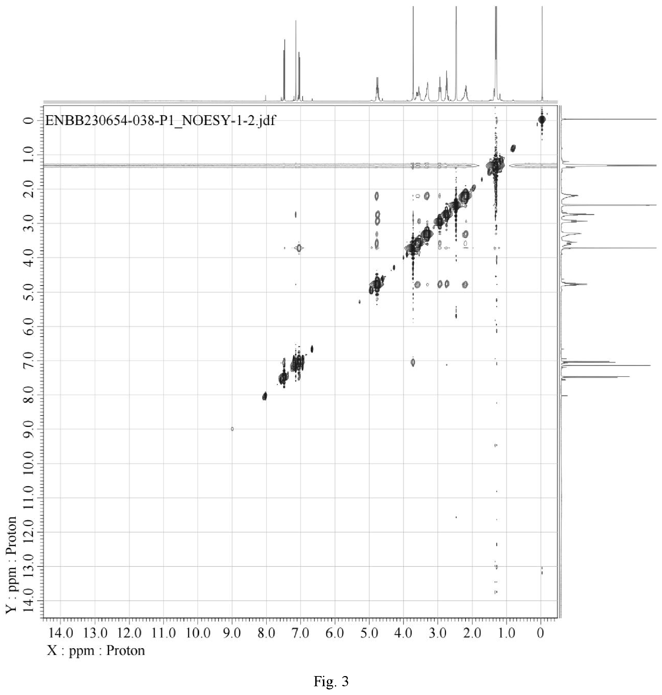

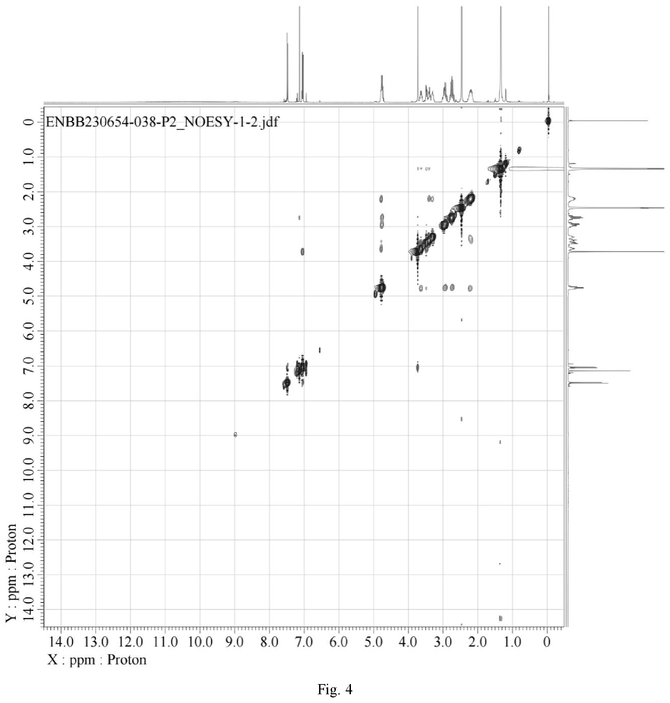

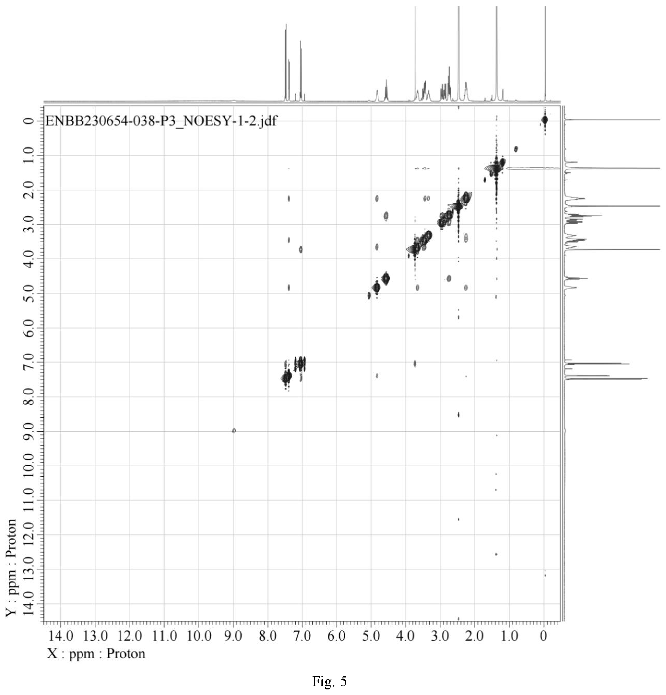

It was confirmed by two-dimensional nuclear magnetic resonance NOESY that Examples 3 and 4 (Figs. 3 and 4) were Compounds 7 and 8, and Example 5 (Fig. 5) was Compound 9.

### Examples 6-7: 3,4-Dichloro-2-((R)-1-((R)-pyrrolidin-3-yl)-1,4,5,6-tetrahydrocyclopenta[c]pyrazol-5-yl)phenol and 3,4-dichloro-2-((S)-1-((R)-pyrrolidin-3-yl)-1,4,5,6-tetrahydrocyclopenta[c]pyrazol-5-yl)phenol

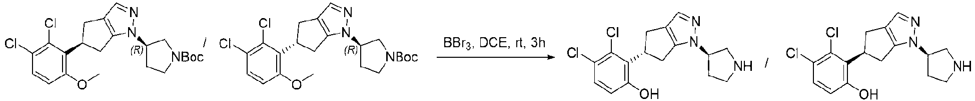

At room temperature, boron tribromide (83.3 mg, 0.33 mmol) was added to a solution of Example 3 (30.0 mg, 0.067 mmol) in dichloroethane (3.0 mL). The mixture was stirred at room temperature for 2 h. After completion of the reaction, the reaction was quenched with ice water (10.0 mL), and the pH was adjusted to about 7 with a saturated aqueous sodium bicarbonate solution. The resulting mixture was extracted twice with ethyl acetate (10.0 mL). The organic phases were combined, dried over anhydrous sodium sulfate, and the solvent was evaporated to dryness by rotary evaporation under reduced pressure, and the crude product was subjected to preparative chromatography to give a white solid as Example 6 (3.5 mg, yield: 16%).

LC-MS: [M+H]+: 338.1.

1H NMR (400 MHz, DMSO-d6) δ 10.40 (s, 1H), 9.14 (s, 1H), 7.34 (d, J = 8.8 Hz, 1H), 7.24 (s, 1H), 6.87 (d, J = 8.8 Hz, 1H), 5.03 - 4.97 (m, 1H), 4.78 - 4.70 (m, 1H), 3.63 - 3.53 (m, 2H), 3.44 - 3.30 (m, 2H), 3.14 (dd, J = 14.8, 8.8 Hz, 1H), 3.00 - 2.90 (m, 2H), 2.73 (dd, J = 13.6, 9.1 Hz, 1H), 2.34 (dd, J = 13.6, 7.1 Hz, 1H), 2.21 - 2.14 (m, 1H).

Referring to the synthesis of Example 6, Example 7 was obtained by replacing Example 3 with Example 4.

LC-MS: [M+H]+: 338.1.

1H NMR (400 MHz, DMSO-d6) δ 10.37 (s, 1H), 9.09 (s, 1H), 7.35 (d, J = 8.8 Hz, 1H), 7.25 (s, 1H), 6.87 (d, J = 8.8 Hz, 1H), 5.02 - 4.96 (m, 1H), 4.78 - 4.70 (m, 1H), 3.59 (dd, J = 12.3, 6.3 Hz, 1H), 3.52 - 3.42 (m, 2H), 3.34 (d, J = 4.7 Hz, 1H), 3.14 (dd, J = 14.8, 8.7 Hz, 1H), 3.01 - 2.91 (m, 2H), 2.74 (dd, J = 13.5, 9.2 Hz, 1H), 2.36 (dd, J = 13.7, 7.3 Hz, 1H), 2.20 (d, J = 5.1 Hz, 1H).

### Examples 8-9: 3,4-Dichloro-2-((S)-2-((S)-pyrrolidin-3-yl)-2,4,5,6-tetrahydrocyclopenta[c]pyrazol-5-yl)phenol and 3,4-dichloro-2-((R)-2-((S)-pyrrolidin-3-yl)-2,4,5,6-tetrahydrocyclopenta[c]pyrazol-5-yl)phenol

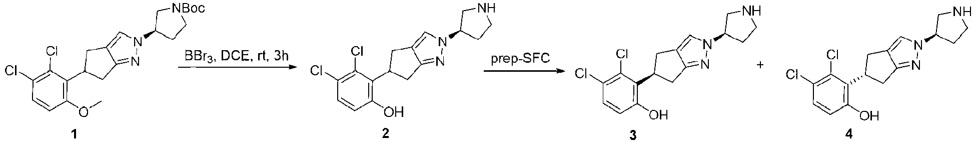

### Synthesis of 3,4-dichloro-2-(2-((R)-pyrrolidin-3-yl)-2,4,5,6-tetrahydrocyclopenta[c]pyrazol-5-yl)phenol (2):

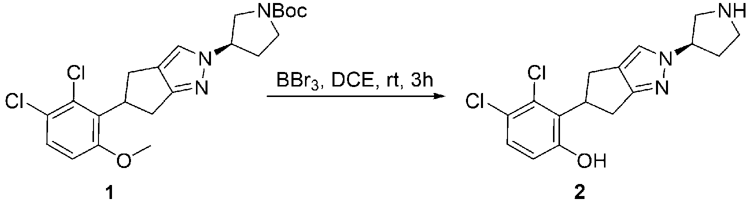

At room temperature, boron tribromide (194.4 mg, 0.78 mmol) was added to a solution of tert-butyl (3R)-3-(5-(2,3-dichloro-6-methoxyphenyl)-5,6-dihydrocyclopenta[c]pyrazol-2(4H)-yl)pyrrolidin-1-carboxylate (70.0 mg, 0.155 mmol) in dichloroethane (7.0 mL). The mixture was stirred at room temperature for 3 h. After completion of the reaction, the reaction was quenched with ice water (10.0 mL), and the pH was adjusted to about 7 with a saturated aqueous sodium bicarbonate solution. The resulting mixture was extracted twice with ethyl acetate (10.0 mL). The organic phases were combined, dried over anhydrous sodium sulfate, and the solvent was evaporated to dryness by rotary evaporation under reduced pressure, and the crude product was subjected to preparative chromatography to give a white solid (20.0 mg, yield: 38%).

LC-MS: [M+H]+: 338.1.

### Preparation of 3,4-dichloro-2-((S)-2-((S)-pyrrolidin-3-yl)-2,4,5,6-tetrahydrocyclopenta[c]pyrazol-5-yl)phenol and 3,4-dichloro-2-((R)-2-((S)-pyrrolidin-3-yl)-2,4,5,6-tetrahydrocyclopenta[c]pyrazol-5-yl)phenol (3/4):

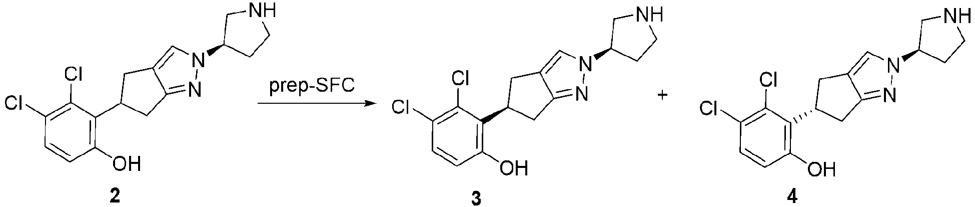

3,4-Dichloro-2-(2-((R)-pyrrolidin-3-yl)-2,4,5,6-tetrahydrocyclopenta[c]pyrazol-5-yl)phenol (20.0 mg, 0.059 mmol) was subjected to preparative chiral chromatography (column: CHIRALPAK AD-H 250 mm 20 mm, 5 µm, mobile phase: 40% EtOH (NH4OH 0.2%)) to give a pair of enantiomers.

The first compound obtained from the SFC resolution was designated as Example 8 (6.9 mg, yield: 35%), with an RT of 2.92 min under the analytical SFC conditions.

LC-MS: [M+H]+: 338.0.

1H NMR (400 MHz, DMSO-d6) δ 10.30 (s, 1H), 9.07 (s, 2H), 7.51 (s, 1H), 7.33 (d, J = 8.8 Hz, 1H), 6.86 (d, J = 8.8 Hz, 1H), 5.10 (s, 1H), 4.56 (t, J = 9.2 Hz, 1H), 3.58 (d, J = 5.0 Hz, 2H), 3.51 - 3.38 (m, 2H), 3.10 (dd, J = 14.8, 9.3 Hz, 1H), 3.00 (dd, J = 14.3, 9.0 Hz, 1H), 2.78 (dt, J = 14.3, 9.6 Hz, 2H), 2.41 - 2.31 (m, 1H), 2.17 (dd, J = 8.5, 4.4 Hz, 1H).

The second compound obtained from the SFC resolution was designated as Example 9 (6.8 mg, yield: 34%), with an RT of 3.88 min under the analytical SFC conditions.

LC-MS: [M+H]+: 338.0.

1H NMR (400 MHz, DMSO-d6) δ 10.32 (s, 1H), 9.07 (s, 1H), 7.51 (s, 1H), 7.34 (d, J = 8.8 Hz, 1H), 6.86 (d, J = 8.8 Hz, 1H), 5.09 (s, 1H), 4.56 (dd, J = 18.4, 9.2 Hz, 1H), 3.62 - 3.50 (m, 1H), 3.49 - 3.36 (m, 2H), 3.11 (dd, J = 14.8, 9.4 Hz, 1H), 3.00 (dd, J = 14.2, 9.0 Hz, 1H), 2.82 - 2.73 (m, 2H), 2.38 (dd, J = 13.7, 7.4 Hz, 1H), 2.27 (d, J = 4.3 Hz, 1H).

Example 10: The following compounds were prepared according to the synthetic routes and the preparation methods for Examples 1-9.

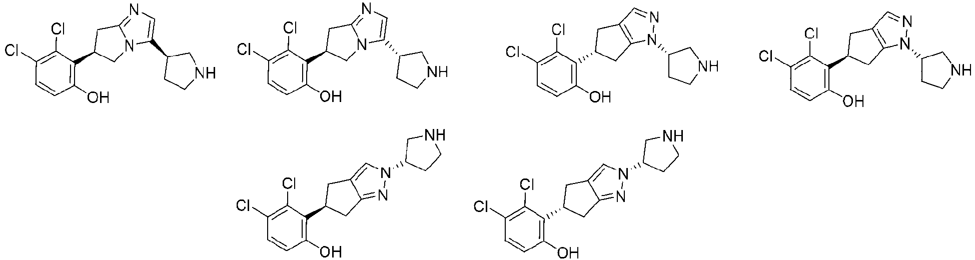

### Example 11: Activity assay

### 1. Assay for inhibitory activity of compounds against Kv1.X

### 1.1. Cell culture

HEK293 cells stably expressing Kv1.X (Kv1.1, Kv1.2, Kv1.3, Kv1.4, Kv1.5) potassium channels were cultured in a DMEM medium containing 10% fetal bovine serum and 800 µg/mL G418 (geneticin) at a culture temperature of 37 °C and a carbon dioxide concentration of 5%.

### 1.2. Solutions used for recording

The extracellular solution contained 40 mM NaCl, 3.5 mM KCl, 1 mM MgCl2•6H2O, 2 mM CaCl2•2H2O, 10 mM D-glucose, 10 mM HEPES, 1.25 mM NaH2PO4•2H2O, and the pH was adjusted to 7.4 with NaOH. The intracellular solution contained 20 mM KCl, 115 mM K-Aspartic, 1 mM MgCl2•6H2O, 5 mM EGTA, 10 mM HEPES, 2 mM Na2-ATP, and the pH was adjusted to 7.2 with KOH.

### 1.3. Patch-clamp testing

The voltage stimulation protocol for recording whole-cell Kv1.X potassium currents via patch-clamp is as follows: After formation of a whole-cell seal, the cell membrane voltage was clamped at -80 mV, and then stepped to +40 mV for 500 ms. Data were acquired repeatedly every 30 s and the effect of the drug on Kv1.X current was observed. The test data were acquired by the EPC-10 amplifier (HEKA) and stored in the PatchMaster (HEKA) software.

Recording electrodes were drawn from capillary glass tubes using a microelectrode puller. Under an inverted microscope, the recording electrode was brought into contact with the cell surface by manipulating a microelectrode manipulator. Negative-pressure suction was applied to form a GΩ seal. After formation of the GΩ seal, a rapid capacitance compensation was given. The application of negative pressure was continued to rupture the cell membrane, forming a whole-cell recording mode. Then, a slow capacitance compensation was given, and the membrane capacitance and series resistance were recorded. No current leakage compensation was provided.

Once the whole-cell recorded Kv1.X current stabilized, drug administration was started. Each drug concentration was allowed to act for 5 min (or until the current reached a steady state) before the next concentration was tested. Multiple concentrations were tested for each test compound. The cover glass plated with the cells was placed in a recording chamber of the inverted microscope. The test compound and the compound-free extracellular solution sequentially flowed through the recording chamber from low concentrations to high concentrations using a gravity perfusion method to act on the cells, and liquid exchange was performed using a vacuum pump during recording. For each cell, the current detected in the compound-free extracellular solution was used as its own control group. The test was independently repeated three times using at least three cells per concentration. All electrophysiological experiments were performed at room temperature.

### 1.4. Data analysis

### (1) Kv1.1, Kv1.2, Kv1.5 - Steady state current

First, the current elicited by each drug concentration was normalized to the current of the blank control ( $\frac{Steady state {current}_{compound}}{Steady state {current}_{control}}$), and then the inhibition rate corresponding to each drug concentration was calculated ( $1 - \frac{Steady state {current}_{compound}}{Steady state {current}_{control}}$ ).

### (2) Kv1.3, Kv1.4 - Peak current

First, the peak current elicited by each drug concentration was normalized to the peak current of the blank control ( $\frac{Peak {current}_{compound}}{Peak {current}_{control}}$), and then the inhibition rate corresponding to each drug concentration was calculated ( $1 - \frac{Peak {current}_{compound}}{Peak {current}_{control}}$). Subsequently, the mean, standard deviation (SD), and standard error (SE) were calculated for the inhibition rate corresponding to each concentration.$Y = Bottom + \left(Top-Bottom\right) / \left(1+10\left(\left(LogIC50-X\right)\times HillSlope\right)\right)$

Using the above equation, the IC50 value for each compound was calculated, and the dose-dependent effect was subjected to non-linear fitting, where IC50 is the half maximal inhibitory concentration. IC50 calculation and curve fitting were performed using the GraphPad Prism software.

### 2. Assay for inhibitory activity of compounds against hERG

### 2.1. Cell culture

CHO cells stably expressing hERG were cultured in 35-mm diameter cell culture dishes at 37 °C in a 5% CO2 incubator. The cells were passaged at a ratio of 1:5 every 48 h. The medium recipe is as follows: 90% F12 (Invitrogen), 10% fetal bovine serum (Gibco), 100 µg/mL G418 (Invitrogen), and 100 µg/mL Hygromycin B (Invitrogen). On the day of the test, the cell culture medium was aspirated, and the cells were rinsed once with the extracellular solution. Then, a 0.25% Trypsin-EDTA solution (Invitrogen) was added, and the cells were digested at room temperature for 3-5 min. The digestion solution was then aspirated, and the cells were resuspended in the extracellular solution and transferred to an experimental dish for electrophysiological recording for later use.

### 2.2. Electrophysiological recording

hERG potassium channel currents were recorded at room temperature by the whole-cell voltage-clamp technique in the CHO cells stably expressing hERG potassium channels. Glass microelectrodes were formed by pulling a glass electrode blank (BF150-86-10, Sutter) using a puller. The tip resistance after perfusion with the electrode internal solution was about 2-5 MΩ. The glass microelectrodes could be connected to a patch clamp amplifier after being inserted into amplifier probes. The clamping voltage and data recording were controlled and recorded by a computer with the pClamp software, with a sampling frequency of 10 kHz and a filtering frequency of 2 kHz. After achieving the whole-cell recording, the cells were clamped at -100 mV. The step voltage evoking hERG potassium current (IhERG) was a 2 s depolarization voltage from -100 mV to +20 mV, followed by repolarization to -50 mV for 1 s, and then returned to -100 mV. This voltage stimulation was applied every 5 s, and after the hERG potassium currents were determined to be stable (for 1 min), the drug application was started. Each test concentration of the compound was applied for at least 1 min (to reach a steady-state effect) or for a maximum of 3 min. Each concentration was tested in at least 2 cells(n ≥ 2).

### 2.3. Data processing

Data analysis and processing were performed using the pClamp, GraphPad Prism 8, and Excel software. The degree of inhibition of the hERG potassium current (the peak hERG tail current evoked at -50 mV) by different compound concentrations was calculated using the following formula:
$Inhibition % = \left[1-\left(I/Io\right)\right] \times 100 %$
where Inhibition % represents the percentage of inhibition of the hERG potassium current by the compound, and I and Io represent the amplitudes of the hERG potassium current after and before drug application, respectively.

The IC50 of the compound was calculated using the GraphPad Prism 8 software by equation fitting as follows:
$Y = Bottom + \left(Top-Bottom\right) / \left(1+{10}^{∧}\left(\left(LogIC50-X\right)\times HillSlope\right)\right)$
where X is the Log value of the test concentration of the test sample, Y is the inhibition percentage at the corresponding concentration, and Bottom and Top are the minimum and maximum inhibition percentages, respectively.

### 3. Experimental results

The compounds of the invention exhibit inhibitory activity against the Kv1.3 potassium channel, with IC50 values less than 20 nM, preferably less than 10 nM, as specifically shown in Table 1 below.

**Table 1. IC50 (nM) values of compounds of the invention for Kv1.3**

| Compound No. | Kv1.3 | Compound No. | Kv1.3 |
|---|---|---|---|
| Example 1 | 0.48 | Example 2 | 1.40 |
| Example 6 | 3.81 | Example 7 | 18.7 |
| Example 8 | 6.63 | Example 9 | 7.46 |

The compounds exhibit no inhibitory activity against the Kv1.1, Kv1.2, Kv1.4, Kv1.5, and hERG channels, with IC50 values all greater than 10 µM, as specifically shown in Table 2 below.

**Table 2. IC50 (nM) values of the compounds of the invention for inhibition of Kv1.1, Kv1.2, Kv1.4, Kv1.5, and hERG**

| Compound No. | Kv1.1 | Kv1.2 | Kv1.4 | Kv1.5 | hERG |
|---|---|---|---|---|---|
| Example 1 | 81490 | >100000 | 1220 | >100000 | 30690 |

Therefore, the compounds of the invention are selective Kv1.3 inhibitors, demonstrating not only excellent activity but also excellent selectivity.

The embodiments of the present invention have been described above. However, the present invention is not limited to these embodiments. Any modification, equivalent replacement, improvement, and the like made without departing from the spirit and principle of the present invention shall all fall within the protection scope of the invention.

## Claims

1. A compound of Formula (A) or a stereoisomer thereof, or a pharmaceutically acceptable salt or deuterated form thereof: 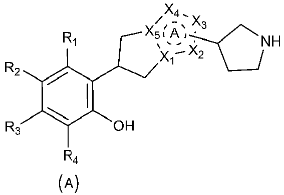 wherein
X₁, X₂, X₃, X₄, and X₅ are each independently selected from C and N, and two of them are N, such that A is a 5-membered heteroaryl group containing two N atoms;
R₁, R₂, R₃, and R₄ are each independently selected from H, F, Cl, Br, CN, C₁₋₄ linear or branched alkyl, C₂₋₄ linear or branched alkenyl, C₂₋₄ linear or branched alkynyl, C₁₋₄ linear or branched alkyloxy, C₂₋₄ linear or branched alkenyloxy, C₂₋₄ linear or branched alkynyloxy, and C₃₋₇ cycloalkyl;
preferably, in Formula (A):
R₁, R₂, R₃, and R₄ are each independently selected from H, F, Cl, Br, CN, C₁₋₄ linear or branched alkyl, C₂₋₄ linear or branched alkenyl, and C₂₋₄ linear or branched alkynyl;
preferably, in Formula (A):
R₁, R₂, R₃, and R₄ are each independently selected from H, F, Cl, and Br;
preferably, in Formula (A):
R₁ and R₂ are each independently selected from F, Cl, and Br, and R₃ and R₄ are H;
preferably, in Formula (A):
X₁ and X₄ are N, and X₂, X₃, and X₅ are C;
preferably, in Formula (A):
X₂ and X₃ are N, and X₁, X₄, and X₅ are C;
preferably, in Formula (A):
X₁ and X₄ are N, and X₂, X₃, and X₅ are C;
R₁ and R₂ are each independently selected from F, Cl, and Br, and R₃ and R₄ are H;
preferably, in Formula (A):
X₂ and X₃ are N, and X₁, X₄, and X₅ are C;
R₁ and R₂ are each independently selected from F, Cl, and Br, and R₃ and R₄ are H.

2. The compound or the stereoisomer thereof, or the pharmaceutically acceptable salt or deuterated form thereof according to claim 1, wherein the compound has a structure of Formula (I), 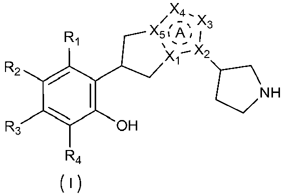 wherein
X₁, X₂, X₃, X₄, and X₅ are each independently selected from C and N, and two of them are N, such that A is a 5-membered heteroaryl group containing two N atoms;
R₁, R₂, R₃, and R4 are each independently selected from H, F, Cl, Br, CN, C₁₋₄ linear or branched alkyl, C₂₋₄ linear or branched alkenyl, C₂₋₄ linear or branched alkynyl, C₁₋₄ linear or branched alkyloxy, C₂₋₄ linear or branched alkenyloxy, C₂₋₄ linear or branched alkynyloxy, and C₃₋₇ cycloalkyl.

3. The compound or the stereoisomer thereof, or the pharmaceutically acceptable salt or deuterated form thereof according to claim 2, wherein
R₁, R₂, R₃, and R₄ are each independently selected from H, F, Cl, Br, CN, C₁₋₄ linear or branched alkyl, C₂₋₄ linear or branched alkenyl, and C₂₋₄ linear or branched alkynyl;
preferably, R₁, R₂, R₃, and R₄ are each independently selected from H, F, Cl, and Br.

4. The compound or the stereoisomer thereof, or the pharmaceutically acceptable salt or deuterated form thereof according to claim 3, wherein
X₁ and X₄ are N, and X₂, X₃, and X₅ are C;
R₁ and R₂ are each independently selected from F, Cl, and Br, and R₃ and R₄ are H.

5. The compound or the stereoisomer thereof, or the pharmaceutically acceptable salt or deuterated form thereof according to claim 3, wherein
X₂ and X₃ are N, and X₁, X₄, and X₅ are C;
R₁ and R₂ are each independently selected from F, Cl, and Br, and R₃ and R₄ are H.

6. The compound or the stereoisomer thereof, or the pharmaceutically acceptable salt or deuterated form thereof according to claim 1, wherein the compound has a structure of Formula (II), 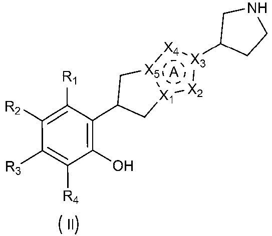 wherein
X₁, X₂, X₃, X₄, and X₅ are each independently selected from C and N, and two of them are N, such that A is a 5-membered heteroaryl group containing two N atoms;
R₁, R₂, R₃, and R₄ are each independently selected from H, F, Cl, Br, CN, C₁₋₄ linear or branched alkyl, C₂₋₄ linear or branched alkenyl, C₂₋₄ linear or branched alkynyl, C₁₋₄ linear or branched alkyloxy, C₂₋₄ linear or branched alkenyloxy, C₂₋₄ linear or branched alkynyloxy, and C₃₋₇ cycloalkyl;
preferably, in Formula (II):
R₁, R₂, R₃, and R₄ are each independently selected from H, F, Cl, Br, CN, C₁₋₄ linear or branched alkyl, C₂₋₄ linear or branched alkenyl, and C₂₋₄ linear or branched alkynyl;
preferably, in Formula (II):
R₁, R₂, R₃, and R₄ are each independently selected from H, F, Cl, and Br;
preferably, in Formula (II):
X₂ and X₃ are N, and X₁, X₄, and X₅ are C;
R₁ and R₂ are each independently selected from F, Cl, and Br, and R₃ and R₄ are H.

7. The compound or the stereoisomer thereof, or the pharmaceutically acceptable salt or deuterated form thereof according to claim 1, selected from the following compounds or pharmaceutically acceptable salts or deuterated forms thereof:

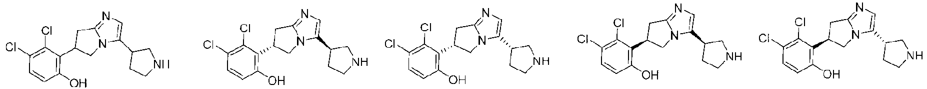

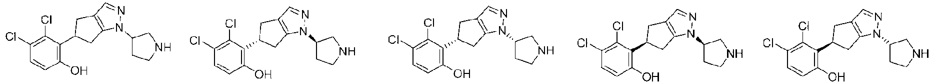

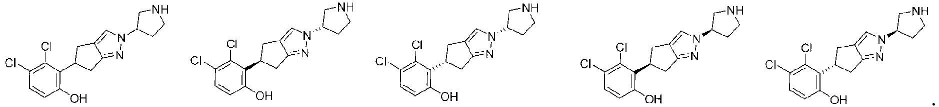

8. A pharmaceutical composition, **characterized by** comprising the compound or the stereoisomer thereof, or the pharmaceutically acceptable salt or deuterated form thereof according to any one of claims 1-7, and a pharmaceutically acceptable carrier.

9. A method for preventing and/or treating a Kv1.3-associated disease, **characterized by** comprising administering to an individual in need thereof a therapeutically and/or prophylactically effective amount of the compound or the stereoisomer thereof, or the pharmaceutically acceptable salt or deuterated form thereof according to any one of claims 1-7, or the pharmaceutical composition according to claim 8, wherein preferably, the disease includes any of a group of immune and inflammatory diseases, such as multiple sclerosis, inflammatory bowel disease, ulcerative colitis, Crohn's disease, rheumatoid arthritis, type 1 diabetes, psoriasis and asthma, spondylitis, and periodontitis, as well as obesity, type 2 diabetes, renal fibrosis, Alzheimer's disease, ischemic stroke, and the like.

10. Use of the compound or the stereoisomer thereof, or the pharmaceutically acceptable salt or deuterated form thereof according to any one of claims 1-7 in preparing a medicament for preventing and/or treating a Kv1.3-associated disease, wherein
preferably, the disease includes any of a group of immune and inflammatory diseases, such as multiple sclerosis, inflammatory bowel disease, ulcerative colitis, Crohn's disease, rheumatoid arthritis, type 1 diabetes, psoriasis and asthma, spondylitis, and periodontitis, as well as obesity, type 2 diabetes, renal fibrosis, Alzheimer's disease, ischemic stroke, and the like.
